(19) Europäisches Patentamt
European Patent Office
Office européen des brevets

(11) Numéro de publication: **0 117 861**
**B1**

# (12) FASCICULE DE BREVET EUROPEEN

(45) Date de publication du fascicule du brevet: **09.12.87**

(51) Int. Cl.⁴: **C 07 D 519/04**

(21) Numéro de dépôt: **84870018.3**

(22) Date de dépôt: **09.02.84**

## (54) Procédé d'obtention de la vincristine.

(30) Priorité: **10.02.83 LU 84640**

(43) Date de publication de la demande: **05.09.84 Bulletin 84/36**

(45) Mention de la délivrance du brevet: **09.12.87 Bulletin 87/50**

(84) Etats contractants désignés: **AT BE CH DE FR GB IT LI NL SE**

(56) Documents cités:
**EP - A - 0 018 231**
**EP - A - 0 037 289**
**EP - A - 0 037 290**

**CHEMICAL ABSTRACTS, vol. 94, 1981, page 689, no. 83738m, Columbus, Ohio, USA, H.J. SCHMIDT et al.: "Oxidation of amines with benzyl(triethyl)ammonium permanganate"**

**Le dossier contient des informations techniques présentées postérieurement au dépôt de la demande et ne figurant pas dans le présent fascicule.**

(73) Titulaire: **OMNICHEM Société anonyme, 12 Avenue de Broqueville, B-1150 Bruxelles (BE)**

(72) Inventeur: **Hannart, Jean, Avenue d'El Pirere, 25, B-1302 Dion Valmont (BE)**

(74) Mandataire: **Van Malderen, Michel et al, p.a. Freylinger & Associés 22 avenue J.S. Bach (bte 43), B-1080 Bruxelles (BE)**

EP 0 117 861 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne un nouveau procédé d'obtention de la vincristine par oxydation sélective du groupe N-méthyle de la vinblastine. La vincristine est un alcaloïde bis-indolique répondant à la formule I dans laquelle R représente un groupe formyle.

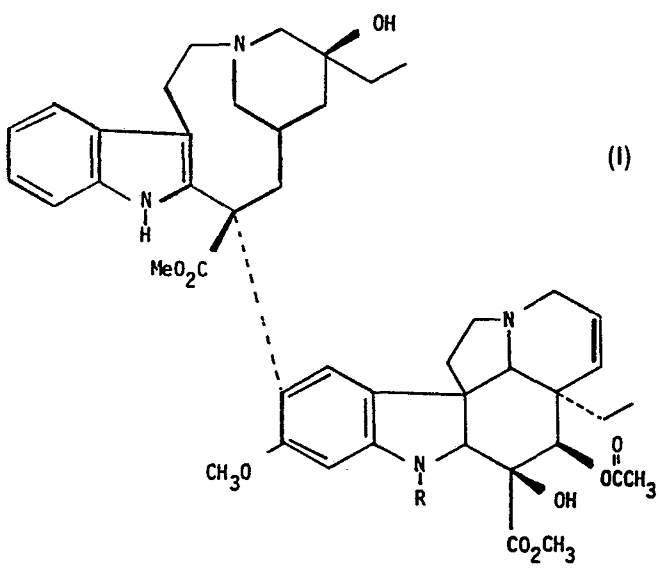

(I)

La vincristine est utilisée en chimiothérapie anticancéreuse, en particulier pour le traitement de certaines leucémies aigües.

Cet alcaloïde est obtenu principalement par extraction à partir de feuilles de Catharanthus Roseus (brevet des Etats-Unis no 3.205.220) où il se trouve accompagné d'autres alcaloïdes bis-indoliques, en particulier la vinblastine. La vinblastine (I, R = $CH_3$) est cependant présente à une concentration très supérieure à celle de la vincristine et constitue donc un précurseur de choix pour l'hémisynthèse de cette dernière.

Plusieurs procédés de fabrication de la vincristine à partir de la vinblastine ont été divulgués. Nous relevons notamment les brevets ou demandes de brevets suivants:

a) Brevet belge 739.337 (Richter Gedeon) qui décrit une méthode d'oxydation de la vinblastine en vincristine par un mélange acide chromique, acide acétique et acétone.

b) Brevet belge 823.560 (Richter Gedeon): l'oxydation est effectuée par l'oxygène dans l'acide formique et en présence d'un catalyseur à base de platine, à température ambiante.

c) Demande de brevet européen 18.231 (Richter Gedeon) : l'oxydation est effectuée par l'acide chromique ou un dichromate de métal alcalin en présence d'anhydride acétique et, éventuellement, d'éthanol et d'un solvant organique immiscible à l'eau.

d) Demande de brevet européen 37.289 (Eli Lilly): l'oxydation est effectuée par le perchlorate de fer (II) en présence de peroxide d'hydrogène et d'acétonitrile.

De plus, la demande de brevet européen 37.290 décrit un procédé d'oxydation de la vinblastine base avec $Na_2Cr_2O_7$ en présence d'acide sulfurique dans le tétrahydrofuranne. Cette réaction, conduite à -50°C, est effectuée avec un rendement de 80-92% calculé par dosage.

Les rendements observés ou la pureté des produits obtenus caractérisant les procédés décrits ci-dessus constituent cependant des désavantages importants.

Un produit secondaire fréquemment formé est la N-déméthyl vinblastine qu'il faut alors reformuler pour obtenir la vincristine.

Le procédé de la présente invention permet de produire la vincristine de manière simple, en quantité importante et à un degré de pureté n'exigeant peu ou pas de purification supplémentaire par recristallisation ou chromatographie.

Le réactif d'oxydation utilisé est l'ion permanganate solubilisé dans le toluène ou le dichlorométhane comme solvant. Une alternative consiste à immobiliser l'anion permanganate sur un résine, par exemple un polymère du type polystyrène comprenant des groupes ammoniums. La solubilisation peut être effectuée par l'action d'un agent complexant du type éther-couronne («crown-ether») sur le permanganate de potassium.

L'anion permanganate peut également être solubilisé en préparant un sel d'ammonium ou de phosphonium quaternaire correspondant qui est soluble dans le dichlorométhane ou le toluène. Dans ce but, on utilisera de préférence le permanganate de benzyltriéthylammonium.

L'obtention de vincristine à partir de la vinblastine en utilisant un sel de permanganate est inattendue dans la mesure où le permanganate de potassium utilisé dans l'acétone oxyde certains dérivés de la vinblastine au niveau de la partie velbanamine de la molécule (Kutney, Balsevich et

Worth, Heterocycles, 11, 69, 1978). Le groupe N-méthyl de la partie vindoline reste intact.

La formation d'un groupe N-CHO indolinique sur un squelette bis-indole du groupe de la vinblastine en utilisant un sel de permanganate n'a jamais été signalée.

Selon un mode du procédé de la présente invention, la vinblastine, de préférence sous la forme de sulfate, est traitée en présence d'un acide organique tel l'acide acétique, avec un excès de permanganate de potassium dissous dans le dichlorométhane ou le toluène en présence de «18-crown-6» éther ou les dérivés dibenzo- ou dicyclohexylcorrespondants. La réaction est menée à une température comprise entre -40°C et -75°C et est avantageusement suivie en chromatographie sur couche mince. Le temps de réaction varie généralement de 5 minutes à 3 heures.

Le permanganate de potassium est de préférence dissous dans le dichlorométhane et la réaction d'oxydation est alors effectuée à -70°C.

La solubilité du permanganate de potassium est en effet substantiellement augmentée en présence d'un polyéther macrocyclique tel que le «18-crown-6« éther (1, 4, 7, 10, 13, 16-hexaoxacyclooctadécane) ou le dérivé dibenzo- ou dicyclohexyl correspondant.

Le mélange réactionnel est ensuite simultanément traité par un réducteur doux et alcalinisé. Dans ce but, on utilisera de préférence une solution aqueuse de bisulfite, de disulfite ou de métabisulfite de sodium et de l'ammoniaque.

La phase organique est séparée et la phase aqueuse est extraite plusieurs fois par le chlorure de méthylène. Les phases organiques réunies sont concentrées sous vide pour fournir un résidu comportant 80-85% de vincristine base, soit un rendement 90-95%.

Alternativement, on peut procéder à l'extraction du milieu réactionnel après réduction sans procéder à une alcalinisation simultanée. La solution aqueuse acide est alors extraite par le dichlorométhane. Cette voie constitue un procédé original de purification de la vincristine formée dans le milieu réactionnel.

Selon un autre mode de réalisation de la présente invention, la vincristine est obtenue par oxydation de la vinblastine par action d'un permanganate d'ammonium quaternaire. Le cation ammonium est de préférence le groupe benzyltriéthylammonium ou benzyl-triméthyl ammonium (voir p.e. Angew. Chem., Intern. Ed. 13, 170, 1974). La réaction est effectuée en 2 à 6 heures à -60°C dans un solvant inerte dans lequel le sel d'ammonium est soluble et d'un acide, de préférence un acide organique de bas poids moléculaire. Un mélange de dichlorométhane et d'acide acétique glacial peut convenir. Après traitement par un agent réducteur doux en milieu aqueux, la solution acide résultante est extraite par le dichlorométhane, puis la phase organique est alcalinisée par lavage à l'aide d'une solution aqueuse basique et concentrée. La vincristine solvatée est isolée avec un rendement supérieur à 90%.

La dernière variante du procédé de l'invention est particulièrement avantageuse du point de vue économique et technique.

Une purification ou séparation peut être effectuée par cristallisation et chromatographie en utilisant des techniques bien connues, ceci à partir du produit brut de la réaction. Le produit peut également être lyophylisé.

Dans la plupart des cas, la vincristine ainsi obtenue peut être transformée directement en un sel d'addition avec un acide organique ou inorganique, de préférence pharmaceutiquement acceptable. Ce sel sera de préférence un sulfate qui pourrait se présenter sous une forme plus ou moins solvatée ou hydratée.

On peut également préparer la vincristine dissoute dans un solvant physiologiquement acceptable et prête a être injectée.

En particulier, le sulfate de vincristine est obtenu par addition de $H_2SO_4$ à une solution de vincristine base brute ou recristallisée dans l'éthanol, dissous dans un mélange chlorure de méthylène-éthanol anhydre, élimination partielle sous vide du chlorure de méthylène et cristallisation.

Le sulfate de vincristine ainsi obtenu présente un degré de pureté suffisant pour être utilisé comme médicament, en particulier sous forme de soluté injectable.

Exemple 1

Formation de vincristine par oxydation au $KMnO_4$ de la vinblastine.

Une solution de 2,78 g de sulfate de vinblastine dans 330 ml de chlorure de méthylène et 42 ml d'acide acétique est dégazée par barbottage d'argon pendant 30 minutes à température ambiante puis refroidie à -70°C.

On additionne goutte à goutte une solution de 1,07 g de $KMnO_4$ dans 125 ml de chlorure de méthylène et 2,22 g de 1, 4, 7, 10, 13, 16-hexaoxacyclooctadecane (18-crown-6). La réaction est suivie et contrôlée en CCM (plaque de Silice - élution éther 80 - méthanol 20).

Le milieu réactionnel est ensuite versé sur un mélange refroidi et agité (consistance pâteuse) de 220 ml de solution aqueuse de disulfite de sodium à 5% et de 110 ml d'ammoniaque 14 N.

L'émulsion éventuelle due à la présence de dioxyde de manganèse est traitée par filtration du mélange total sur terre de diatomées. Les deux phases claires sont décantées et la phase aqueuse est épuisée par le chlorure de méthylène. Les extraits sont réunis, séchés sur $MgSO_4$ et évaporés à sec (poids = 2,93 g).

Le résidu est dosé à 83% de vincristine base, soit un rendement de 96%.

La vincristine base est obtenue par cristallisation du résidu dans l'éthanol et est indique à un échantillon authentique de référence (spectre de masse, $\alpha_D$, spectre UV, spectre RMN $^1H$).

Sulfate

La vincristine de base est solubilisée dans un mélange de $CH_2CL_2$ et d'éthanol (60:40). Le pH est ajusté à 4 par addition d'une solution à 1,84% d'$H_2SO_4$ dans l'éthanol anhydre.

Le solvant est partiellement éliminé par distillation sous vide et le sulfate cristallise lentement par agitation de la solution à température ambiante.

Exemple 2.

Formation de la vincristine par oxydation de la vinblastine en présence de permanganate de benzyltriéthylammonium.

Dans un ballon de 4 litres, on place 20 g de sulfate de vinblastine dans 1,6 litre de dichlorométhane. On ajoute 360 ml d'acide acétique sec qui solubilise la vinblastine. On refroidit sous atmosphère d'argon à -60°C.

Une solution de 27,68 g de permanganate de benzyltriéthylammonium dans 1,6 litre de dichlorométhane est préparée. Cette solution est filtrée sur papier afin d'éliminer un éventuel précipité de dioxyde de manganèse.

On additionne la solution oxydante goutte à goutte sans dépasser -60°C. L'addition est effectuée en 3 heures. On refroidit jusqu'à état pateux une solution de 3,2 litres de disulfite de sodium à 5%. On transfère, sous pression d'argon, le mélange réactionnel dans la solution de disulfite qui est agitée mécaniquement. On laisse revenir à température ambiante et la phase aqueuse est épuisée par le dichlorométhane (3,2 litres initialements, puis 3 litres, puis deux fois 2 litres).

Les phases organiques réunies sont alcalinisées par addition de 2 litres de bicarbonate de sodium à 80 g/litre. Le dichlorométhane est alors évaporé sous vide. On obtient ainsi 16,47 g de vincristine brute.

Après séchage sous vide pendant 12 heures à température ambiante, on obtient 15,12 g de vincristine de pureté supérieure à 92%.

## Revendications

1. Procédé d'obtention de la vincristine ou de ses sels d'addition à partir de la vinblastine caractérisé en ce que la vinblastine est traitée par l'ion permanganate en milieu acide dans un solvant inerte constitué par le toluène ou par le dichlorométhane, l'ion permanganate étant solubilisé par action d'un poly-éther macrocyclique sur le permanganate de potassium ou sous la forme d'un sel d'ammonium quaternaire soluble dans le solvant inerte susmentionné, et en ce qu'on maintient une température comprise entre -40°C et -75°C dans le milieu réactionnel.

2. Procédé selon la revendication 1 caractérisé en ce que l'acide utilisé est l'acide acétique.

3. Procédé selon la revendication 1 ou 2 caractérisé en ce que la température du milieu réactionnel est maintenue à environ -70°C.

4. Procédé selon la revendication 1 caractérisé en ce que le sel d'ammonium quaternaire est le permanganate de benzyltriéthylammonium.

5. Procédé selon la revendication 1 dans lequel le milieu réactionnel, après traitement au permanganate, est traité par une solution aqueuse de bisulfite.

6. Procédé selon les revendications 1 à 5 dans lequel avant d'isoler la vincristine de manière classique, le milieu réactionnel aqueux résultant de l'addition de la solution de bisulfite, est extrait par le dichlorométhane.

## Patentansprüche

1. Verfahren zur Herstellung von Vincristin oder dessen Additionssalze aus Vinblastin, dadurch gekennzeichnet, dass Vinblastin mit Permanganationen in saurem Medium, in einem inerten Lösungsmittel, das aus Toluol oder Dichlormethan besteht, behandelt wird, wobei die Permanganationen durch Wirkung eines Makrocyclischen Polyäthers auf Kaliumpermanganat löslich gemacht werden oder in der Form eines quaternären in das obengenannte inerte Lösungsmittel löslich gemachten Ammoniumsalzes verwendet werden, und dass eine Temperatur zwischen -40°C und -75°C im Reaktionsmedium gehalten wird.

2. Verfahren nach Anspruch 1, dadurch gekennzeichnet, dass die verwendete Säure aus Essigsäure besteht.

3. Verfahren nach Anspruch 1 oder 2, dadurch gekennzeichnet, dass die Temperatur des Reaktionsmediums bei ungefähr -70°C gehalten wird.

4. Verfahren nach Anspruch 1, dadurch gekennzeichnet, dass das quaternäre Ammoniumsalz aus Benzyltrietyl-ammoniumpermanganat besteht.

5. Verfahren nach Anspruch 1, worin das Reaktionsmedium nach Behandlung mit Permanganat mit einer wässerigen Bisulfitlösung behandelt wird.

6. Verfahren nach den Ansprüchen 1 bis 5, worin, vor der klassischen Abtrennung von Vincristin, das aus der Addition der Bisulfitlösung entstandene wässrige Reaktionsmedium mit Dichlormethan extrahiert wird.

## Claims

1. Process for the preparation of vincristine or its addition salts from vinblastine characterized in that vinblastine is treated with permanganate ion in acid medium in an inert solvent consisting of toluene or dichloromethane, the permanganate ion being solubilized by action of a macrocyclic polyether on the potassium permanganate or in the state of a quaternary ammonium salt soluble in the said inert solvant, and in that the reaction medium is maintained at a temperature of between -40°C and -75°C.

2. Process according to claim 1 characterized in that the acid used is acetic acid.

3. Process according to claim 1 or 2 characterized in that the temperature of the reaction medium is maintained at about -70°C.

4. Process according to claim 1 characterized in that the quarternary ammonium salt is benzyltriethylamonium permanganate.

5. Process according to claim 1 wherein the

reaction medium is treated, after treatment with permanganate, with an aqueous solution of bisulfite.

6. Process according to claims 1 to 5 wherein, before isolating the vincristine in conventional manner, the aqueous reaction medium resulting from the addition of the bisulfite solution is extracted with dichloromethane.